# EUROPEAN PATENT APPLICATION

(11) **EP 4 570 914 A1**
(43) Date of publication of application: **18.06.2025**
(21) Application number: 23852503.4
(22) Date of filing: 04.08.2023
(51) Int. Cl.: C12P 5/02, B01D 53/62, C12M 1/04, C12M 1/107

(54) **DEVICE AND METHOD FOR GENERATING METHANE GAS AND INVOLVING REMOVAL OF CARBON DIOXIDE**

(30) Priority: 08.08.2022 JP 2022126591
(71) Applicant: Ebara Jitsugyo Co. Ltd., Tokyo 104-8174 (JP)
(72) Inventor: Katsu Yosei, Tokyo 104-8174 (JP); TANAKA Toshihiro, Tokyo 104-8174 (JP); KAWASAKI Yu, Tokyo 104-8174 (JP)
(74) Representative: Sonnenberg Harrison Partnerschaft mbB
(86) International application number: PCT/JP2023/028606
(87) International publication number: WO 2024/034541

(57) **Abstract**

Provided are a device and a method that are for generating methane gas, that involve removal of carbon dioxide, and that are capable of efficiently generating high-concentration methane gas even when a microorganism is used. A device for generating methane gas that involves the removal of carbon dioxide where a hydrogen containing gas A and a CO₂ containing gas B are supplied (C) to an anaerobic bioreactor tank 1 so that microbial treatment is carried out to discharge a treated gas that contains methane gas, is characterized in that a fixed bed 20 that is filled in with a carrier to which microorganisms are attached is provided within the reactor tank 1, and a gas circulating line is provided to circulate (D) at least a portion of the treated gas E to the reactor tank.

## Description

### Technical Field

The present invention relates to a device and a method for generating methane gas that involve the removal of carbon dioxide, and in particular, to a device and a method for generating methane gas that involve the removal of carbon dioxide, where microbial treatment is carried out on a hydrogen containing gas and a CO₂ containing gas so as to discharge a treated gas that contains methane gas.

### Background Art

In recent years, the reduction and removal of carbon dioxide, which is a material that causes global warming, have become a major issue. Carbon dioxide is generated from the incineration of fossil fuels, and thus, is required to be reduced, collected, and reused. In addition, carbon dioxide is included in a biogas that has been acquired through methane fermentation of organic sludge such as organic wastewater, sewage sludge, organic waste, and food dregs, which makes it more necessary to remove carbon dioxide.

As for the technology for removing and using carbon dioxide, the methanation technology as shown in the following reaction formula (1) has already been publicly known.

4H₂ + CO₂ → CH₄ + 2H₂O ... (1)

As shown in Patent Literature 1 and 2, a method for removing carbon dioxide and generating methane gas has already been disclosed where a high methane generating capability is achieved by using a catalyst under the conditions of a high pressure and a high temperature. Such a method, however, consumes a large amount of energy, and the operation costs for the generation system and device are high, and the durability of the device is low.

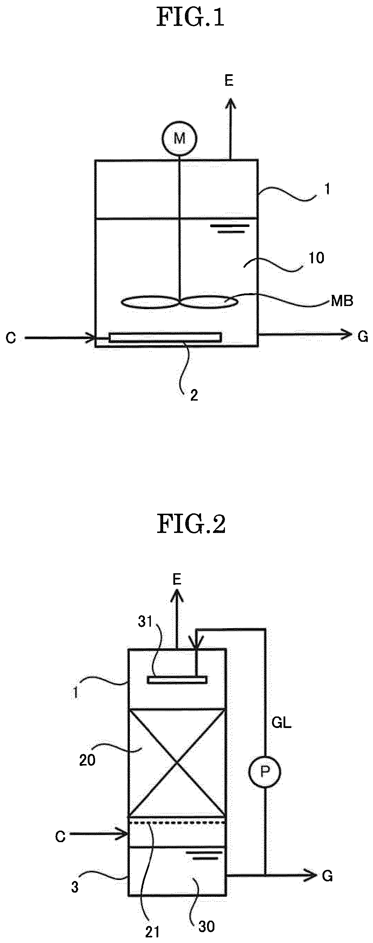

In contrast, as shown in Patent Literature 3, the above-described reaction formula (1) is also made possible due to the action of microorganisms. As shown in FIG. 1, a liquid culture medium 10 that contains microorganisms is put on the inside of a reactor tank 1 into which carbonic acid gas and hydrogen gas are supplied as denoted by the symbol C, and then, air is supplied to the liquid culture medium from an air diffusing means 2 within the reactor tank. In addition, stirring blades MB are driven by a motor M in order to stir the floating microorganisms and the liquid culture medium.

However, the methane generating capability thereof is low and restricted. For example, the amount of methane gas generated per reactor is as low as approximately 10.8 L/L-Reactor/d (Unit: The amount of methane gas generated per liter within a reactor tank in a day) at the maximum, and the concentration of the methane gas in the treated gas that is discharged from the reactor tank is as low as approximately 55 %. The methanation reaction caused by the microorganisms is greatly affected by the concentration of the microorganisms and the treatment conditions, in particular, by the concentration of the dissolution of carbon dioxide and hydrogen gas in the reaction liquid or the dissolution rate and the diffusion rate into the microorganisms. Therefore, stabilizing a high methane generation rate and generating a high concentration methane gas have become major issues with methanation caused by microorganisms.

Patent Literature 3 also proposes a method for manufacturing methane gas by using methane generating bacteria that are fixed to a carrier. For example, as shown in FIG. 2, a fixed bed 20 that is filled in with a carrier to which microorganisms are attached is held within a reactor tank 1 by using a support net 21 or the like. An ejection pipe 31 is arranged in an upper portion of the fixed bed so that a liquid that contains a nutrition source for the microorganisms is supplied to the fixed bed. The liquid that drips from the fixed bed is stored in a liquid storage unit 3 that is arranged in a lower portion of the fixed bed. The symbol 30 denotes the liquid that has been stored in the storage unit 3. The liquid is circulated by means of a pump P through a liquid circulating line GL so as to be resupplied to the fixed bed 20 from the ejection pipe 31.

Carbonic acid gas and hydrogen gas are supplied between the fixed bed 20 and the liquid 30 that has been stored in the liquid storage unit 3 as denoted by the symbol C so as to be treated by microorganisms while passing through the fixed bed 20, and thus, are converted to a treated gas that contains methane gas, and then discharged from the reactor tank 1 as denoted by the symbol E.

In the same manner as in the case of FIG. 1, methane gas generating devices that use such a fixed bed still allow large amounts of carbonic acid gas and hydrogen gas that have not been treated to be included in the treated gas, and therefore, stabilizing a high methane generating rate and generating a high concentration methane gas have become major issues.

### Citation List

### Patent Literature

Patent Literature 1: Japan Patent No. 5562873
Patent Literature 2: Japan Patent No. 6956665
Patent Literature 3: Japanese Examined Patent Publication S63(1988)-49999

### Summary of the Invention

### Technical Problem

An object of the present invention is to solve the above-described problems and to provide a device and a method for generating methane gas that involves the removal of carbon dioxide, which makes it possible to generate a high concentration methane gas at a high efficiency even in the case where microorganisms are used.

### Solution to Problem

In order to achieve the above-described object, the device and the method for generating methane gas that involves the treatment of carbon dioxide according to the present invention have the following features.
(1) A device for generating methane gas that involves the removal of carbon dioxide where a hydrogen containing gas and a CO₂ containing gas are supplied to an anaerobic bioreactor tank so that microbial treatment is carried out to discharge a treated gas that contains methane gas is characterized in that a fixed bed that is filled in with a carrier to which microorganisms are attached is provided within the reactor tank, and a gas circulating line is provided to circulate at least a portion of the treated gas to the reactor tank.
(2) The device for generating methane gas that involves the removal of carbon dioxide according to the above (1) is characterized by having: a liquid supplying means for supplying a liquid that contains a nutrition source for the microorganisms from an upper portion of the fixed bed; a liquid storage unit for storing the liquid that is arranged in a lower portion of the fixed bed; and a liquid circulating line for circulating a portion of the liquid that has been stored in the liquid storage unit to the liquid supplying unit.
(3) The device for generating methane gas that involves the removal of carbon dioxide according to the above (2) is characterized in that hydrogen containing gas is supplied to the liquid storage unit, and CO₂ containing gas is supplied to a space between the fixed bed and the liquid storage unit.
(4) The device for generating methane gas that involves the removal of carbon dioxide according to the above (3) is characterized in that the gas circulating line is connected to the space between the fixed bed and the liquid storage unit.
(5) The device for generating methane gas that involves the removal of carbon dioxide according to the above (4) is characterized in that the fixed bed is formed of a plurality of fixed bed units, and the gas circulating line allows at least a portion of the treated gas to be led out from a portion that connects a certain fixed bed unit to the next fixed bed unit.
(6) The device for generating methane gas that involves the removal of carbon dioxide according to any of the above (1) through (5) is characterized by having an NH₃ removing means for removing NH₃ that is contained in the treated gas at a later stage of the reactor tank.
(7) The device for generating methane gas that involves the removal of carbon dioxide according to any of the above (1) through (5) is characterized in that a gas separating means for separating methane gas that is contained in the treated gas is provided at a later stage of the reactor tank, and the remaining gas from which methane gas has been removed by means of the gas separating means is supplied to the gas circulating line.
(8) The device for generating methane gas that involves the removal of carbon dioxide according to the above (6) is characterized in that a gas separating means for separating methane gas that is contained in the gas that has passed through the NH₃ removing means is provided at a later stage of the NH₃ removing means, and the remaining gas from which methane gas has been removed by the gas separating means is supplied to the gas circulating line.
(9) The device for generating methane gas that involves the removal of carbon dioxide according to any of the above (2) through (5) is characterized by having a means for supplying an alkali chemical in the liquid storage unit or in the middle of the liquid circulating line.
(10) The device for generating methane gas that involves the removal of carbon dioxide according to any of the above (1) through (4) is characterized in that the height of the fixed bed is set in a range from 1 m to 10 m.
(11) The device for generating methane gas that involves the removal of carbon dioxide according to any of the above (1) through (5) is characterized by having a circulation amount adjustment means for setting the amount of circulation of the treated gas that is to be circulated through the gas circulating line is set to 0.5 to 50 times the total amount of the hydrogen containing gas and the CO₂ containing gas that are supplied to the reactor tank.
(12) A method for generating methane gas that involves the removal of carbon dioxide, where a hydrogen containing gas and a CO₂ containing gas are supplied to an anaerobic bioreactor tank so that microbial treatment is carried out within the reactor tank so as to discharge a treated gas that contains methane gas is characterized in that a fixed bed that is filled in with a carrier to which microorganisms are attached is provided within the reactor tank, and at least a portion of the treated gas is circulated to the reactor tank.
(13) The method for generating methane gas that involves the removal of carbon dioxide according to the above (12) is characterized in that a liquid that contains a nutrition source for the microorganisms is supplied from an upper portion of the fixed bed, the liquid is stored in a lower portion of the fixed bed, and a portion of the stored liquid is circulated so as to be supplied to an upper portion of the fixed bed.
(14) The method for generating methane gas that involves the removal of carbon dioxide according to the above (13) is characterized in that hydrogen containing gas is supplied into the stored liquid, and CO₂ containing gas is supplied to a space between the fixed bed and the stored liquid.
(15) The method for generating methane gas that involves the removal of carbon dioxide according to the above (13) is characterized in that the treated gas that is to be circulated is supplied to a space between the fixed bed and the stored liquid.
(16) The method for generating methane gas that involves the removal of carbon dioxide according to any of the above (12) through (15) is characterized in that NH₃ that is contained in the treated gas is removed.
(17) The method for generating methane gas that involves the removal of carbon dioxide according to any of the above (12) through (15) is characterized in that methane gas is separated from the treated gas, and the remaining gas from which the methane gas has been removed is circulated to the reactor tank.
(18) The method for generating methane gas that involves the removal of carbon dioxide according to the above (16) is characterized in that methane gas is separated from the treated gas from which NH₃ has been removed, and the remaining gas from which the methane gas has been removed is circulated to the reactor tank.
(19) The method for generating methane gas that involves the removal of carbon dioxide according to any of the above (12) through (15) is characterized in that an alkali chemical is supplied to either the stored liquid or the circulated liquid.

The method for generating methane gas that involves the removal of carbon dioxide according to any of the above (12) through (15) is characterized in that the ratio of the mass of H₂ in the hydrogen containing gas to be supplied to the anaerobic bioreactor tank to the mass of CO₂ in the CO₂ containing gas is set to a range from 1:5 to 1:11.

### Advantageous Effects of the Invention

According to the present invention, a device and a method for generating methane gas that involves the removal of carbon dioxide where a hydrogen containing gas and a CO₂ containing gas are supplied to an anaerobic bioreactor tank where bacterial treatment is carried out within the reactor tank so as to discharge a treated gas that contains methane gas is provided in such a manner that a fixed bed that is filled in with a carrier to which microorganisms are attached is provided within the reactor tank, and at least a portion of the treated gas is circulated to the reactor tank, which allows the carbon dioxide and hydrogen that remain in the treated gas to be resupplied to the reactor tank so as to be contributed to the methane generating reaction, and thus, it becomes possible to make the concentration of generated methane gas in the treated gas higher and the methane gas conversion rate in the entirety of the process higher.

### Brief Description of the Drawings

FIG. 1 is a diagram showing the device for generating methane gas according to the prior art;
FIG. 2 is a diagram showing an example of a device for generating methane gas using a fixed bed;
FIG. 3 is a diagram showing a basic concept of the device for generating methane gas that involves the removal of carbon dioxide according to the present invention;
FIG. 4 is a diagram showing the first embodiment of the device for generating methane gas that involves the removal of carbon dioxide according to the present invention;
FIG. 5 is a diagram showing the second embodiment of the device for generating methane gas that involves the removal of carbon dioxide according to the present invention;
FIG. 6 is a diagram showing the third embodiment of the device for generating methane gas that involves the removal of carbon dioxide according to the present invention;
FIG. 7 is a diagram showing the fourth embodiment of the device for generating methane gas that involves the removal of carbon dioxide according to the present invention;
FIG. 8 is a diagram showing an example where a hydrogen containing gas and a CO₂ containing gas are supplied to different locations that can be applied to the device for generating methane gas that involves the removal of carbon dioxide according to the present invention;
FIG. 9 is a diagram showing an example where a gas circulating line is provided with the device for generating methane gas in FIG. 8;
FIG. 10 is a diagram showing an example where the fixed bed of the device for generating methane gas in FIG. 8 is formed of a plurality of fixed bed units;
FIG. 11 is a diagram showing an example where a gas circulating line is provided with the device for generating methane gas in FIG. 10;
FIG. 12 is a diagram showing an example of the configuration where sampling a portion of the treated gas and supplying a CO₂ containing gas are carried out between adjacent fixed bed units in the device for generating methane gas in FIG. 11;
FIG. 13 is a diagram showing an example of the application of the device for generating methane gas in FIG. 11, where the circulated gas is directly sampled from within a fixed bed unit;
FIG. 14 is a diagram showing an example of the application of the device for generating methane gas in FIG. 8, where a plurality of fixed bed units is incorporated into different reactor tanks;
FIG. 15 is a diagram showing an example of the application of the device for generating methane gas in FIG. 14, where the circulated gas is introduced into the reactor tank at a later stage so as to stir the liquid in the liquid storage unit;
FIG. 16 is a diagram showing an example where a gas circulating line is provided with the device for generating methane gas in FIG. 14;
FIG. 17 is a diagram showing an example of the application of the device for generating methane gas in FIG. 16, where the circulated gas is introduced into the reactor tank at a later stage so as to stir the liquid in the liquid storage unit;
FIG. 18 is a diagram showing another example where a gas circulating line is provided with the device for generating methane gas in FIG. 14; and
FIG. 19 is a diagram showing an example of the application of the device for generating methane gas in FIG. 18, where the treated gas that is discharged from the reactor tank at a later stage is circulated only in the reactor tank at the later stage.

### Description of Embodiments

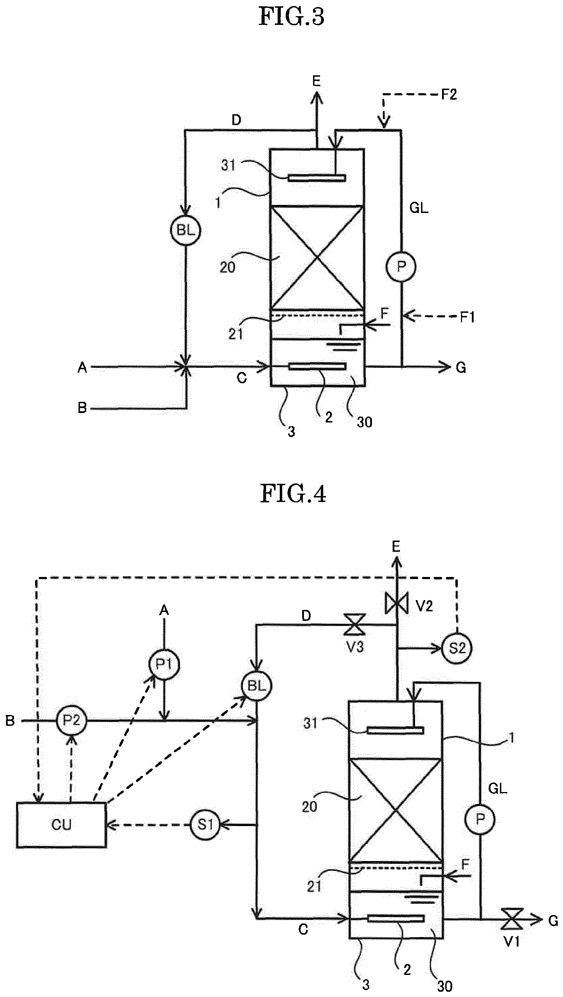

The device and the method for generating methane gas that involves the removal of carbon dioxide according to the present invention is described in detail below in reference to FIGS. 3 through 19. Here, the following description mainly focuses on the device for generating methane gas for the purpose of simplification of the description.

As shown in FIG. 3, the present invention provides a device for generating methane gas that involves the removal of carbon dioxide, where a hydrogen containing gas A and a CO₂ containing gas B are supplied (symbol C) to an anaerobic bioreactor tank 1 for carrying out microbial treatment, from which a methane gas containing treated gas E is discharged, and where the device is characterized in that a fixed bed 20 that is filled in with a carrier to which microorganisms are attached is provided within the reactor tank 1, which has a gas circulating line for circulating (symbol D) at least a portion of the treated gas E to the reactor tank.

There are also some cases where the reactor tank having a fixed bed that is filled in with a carrier to which microorganisms are attached is referred to as an "anaerobic biofilm reactor tank," where a "biofilm" that shows the state of the microorganisms that are attached to the carrier is used.

In addition, there are some cases where the device for generating methane gas using a fixed bed that is filled in with a carrier to which microorganisms are attached is referred to as a "carrier filled type" device for generating methane gas.

It is possible to treat the gas A that has been supplied to the anaerobic bioreactor tank 1 as long as the gas A contains carbon dioxide gas. A biogas that has been acquired through methane fermentation of organic wastewater or organic sludge may be used as the gas A. In addition, carbon dioxide that is generated from incineration in an industrial plant or a thermoelectric power station (exhaust gas) may be used.

A gas that contains hydrogen can be used as the gas B when supplied to the anaerobic bioreactor tank 1. Hydrogen gas that has been acquired through the electrolysis of water by using renewable energy from sunlight or wind power, or by using excess power from a power station during the nighttime may be used as the gas B. Alternatively, hydrogen gas that is generated from a production process in an industrial plant as a byproduct may be used.

It is preferable for the CO₂ containing gas A and the hydrogen containing gas B to not include oxygen because they are supplied to the anaerobic bioreactor tank 1. In addition, as shown in FIG. 3, the method for supplying the gas A and the gas B to the reactor tank 1 includes a method for supplying a mixed gas C, where the gas A and the gas B are mixed, to the reactor tank, and a method for supplying the gas A and the gas B separately as described below.

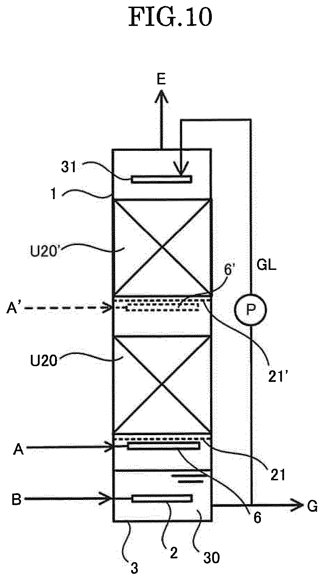

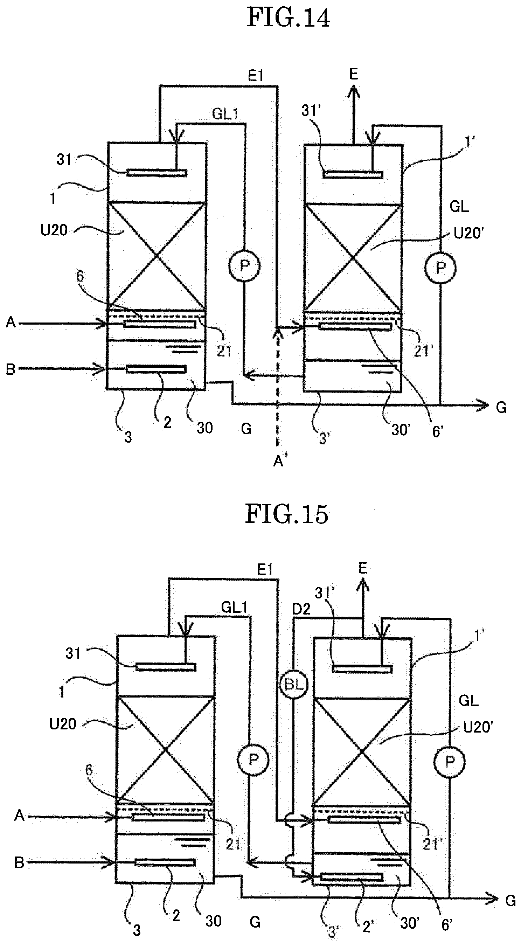

As for the possible ways of supplying the gases A and B into the reactor tank 1, the gases can be supplied between the fixed bed 20 and the liquid storage unit 3 as shown in FIG. 2, or the gas A can be supplied between adjacent fixed bed units, where the fixed bed 20 is divided into a plurality of fixed bed units (U20, U20') as described below (see FIGS. 10 and 14). In addition, as shown in FIG. 3, it is possible to arrange an air diffusing means 2, such as an air diffusing pipe, in the bottom portion of the reactor tank 1 so that air can be diffused in the nutrition source containing liquid 30 that is stored in the liquid storage unit 3. As a result, it also becomes possible to mix and stir the liquid 30 or to convert a portion of the liquid 3 into mist so that a nutrition source can be supplied to the microorganisms within the fixed bed 20 together with the gases A and B. Naturally, an air diffusing means may be provided to correspond to the mixed gas C, or air diffusing means may be provided to correspond to the gases A and B respectively.

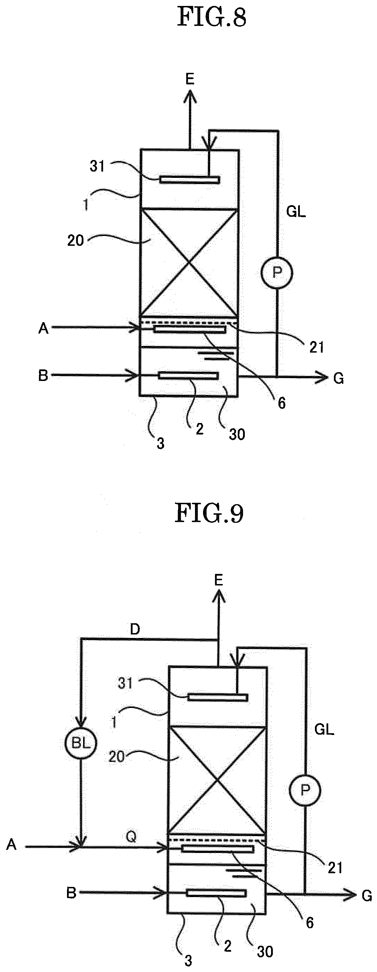

As described in detail in reference to FIGS. 8 and onwards, in the case where the gas A and the gas B are injected into the reactor tank separately, it is preferable for the portion through which the CO₂ containing gas A is injected to be a gas phase that is filled in with a carrier (in particular, a space between the fixed bed 20 and the liquid storage unit 3). It is preferable for the portion through which the hydrogen containing gas B is injected to be in the liquid storage unit 3 for the circulated liquid. It is preferable for both the gas A and the gas B to be supplied through an air diffusing pipe. When the gas is supplied from an air diffusing pipe, the flow of the gas becomes uniform within the reactor tank, which makes the frequency of contact with the carrier uniform, and thus, a high reaction efficiency can be gained. In addition, the air diffusing means 6 for the gas A is arranged in proximity to the lower surface of the fixed bed 20 (support net 21 for supporting the fixed bed) in order to supply the gas A to the gas phase because CO₂ easily dissolves in water.

Here, needless to say, it is possible to use a technology for injecting the gas A and the gas B into the reactor tank separately in the device or the method for generating methane gas even in the case where a gas circulating line is not provided.

It is preferable to use a fine bubble diffusing device having a high efficiency of dissolving gas as the air diffusing means 2. The higher the number of fine bubbles is, the higher the amount of mist can be generated from the liquid 30, which accelerates the methane generating reaction caused by microorganisms.

Any of the fine bubble diffusing devices can be used from among a membrane type, a ceramic air diffusing board type, or an injector type in order to gain similar effects. In the case of a membrane type or a ceramic type, the smaller the diameter of the surface pores thereof is, the smaller the bubbles of the generated gas are, which makes it possible to generate a fine mist, and makes it easier to diffuse the gas into the inside of the fixed bed 20. In addition, in the case where the bubbles of the gas are smaller, a portion of the gas dissolves into the liquid 30 at a higher efficiency, which makes it possible to supply the dissolved gas to the microorganisms together with the liquid 30 when the liquid 30 is circulated so as to be supplied to the fixed bed 20. It is desirable for the diameter of the surface pores to be 30 µm or less.

In the case of an ejector, it is also preferable for the diameter of pores for fine bubbles to be 30 µm or less because fine bubbles are created physically. Likewise, an air diffusing means by which microbubbles or nanobubbles can be gained also allows fine mist to be generated or hydrogen to dissolve at a higher efficiency, and thus, the effects of increasing the efficiency of methane generation can be achieved. In addition, these air diffusion means can be applied as the air diffusing means 6 in FIGS. 8 and onwards.

The fixed bed 20 in the anaerobic bioreactor tank 1 holds microorganisms that are mainly methane generating bacteria for converting carbon dioxide gas and hydrogen gas into methane gas through a reaction.

A bacteria derived from digested sludge can be used as the methane generating bacteria, and it is desirable to use a hydrogen assimilating methane generating bacteria that can generate methane from hydrogen and carbon dioxide. Concretely, "Methanobacterium thermoautotrophicum," "Methanobacterium formicicum," "Methanococas vanielii," and "Methanosaricina barkerii" are appropriately available.

A carrier is used to hold the microorganisms. It is preferable for a hydrophilic carrier, to which a hydrogen assimilating methane generating bacteria can be easily attached, to be used as the carrier. In order to attach the above-described methane generating bacteria to the surface of the carrier stably, it is effective to immerse the carrier in an anaerobic digested sludge in advance. In addition, a hydrophilic carrier, which is a high polymer carrier that cannot be decomposed by microorganisms and to which hydrogen assimilating methane generating bacteria as described above can be easily attached, is preferable as the carrier. As examples of the high polymer carrier, hard carriers made of polyethylene, polyurethane, or polypropylene, and gel carriers made of synthesized high polymers such as polyethylene glycol (PEG), polyvinyl alcohol (PVA), polyacrylamide, polycarbonate, or a photocurable resin, or high polymers such as carrageenan or sodium alginate can be cited.

As for the shape of the carrier, any of a sphere, a square, a cylinder, or a tube can be used, where the effective diameter is 5 mm to 20 mm, preferably 10 mm to 15 mm, so that the carrier can be stably separated from the support net. It is preferable for the specific surface area of the carrier to be 100 m²/m³ to 5000 m²/m³.

Carriers having a large number of fine pores on the surface, carriers of which the inside is hollow, carriers having an infinite number of recesses and protrusions on the surface, and carriers with activated carbon powder being attached allow smooth attachment and fixation of methane generating bacteria in such a manner that high concentration methane generating bacteria can be attached through a domestication culture during a short period of time. Furthermore, methane generating bacteria can be attached and fixed to the surface of a carrier at a high concentration in the reactor tank for over a long period of time, and thus, a stable methane conversion performance can be achieved.

It is preferable for the specific gravity of the carrier to be close to 0.9 to 1.0 so as not to be an overload even when the bioreactor tank is filled to the full. The height of the carrier layer (fixed bed) with which the reactor tank is filled (the thickness in the direction of the height) is 1 m to 10 m, preferably 3 m to 5 m.

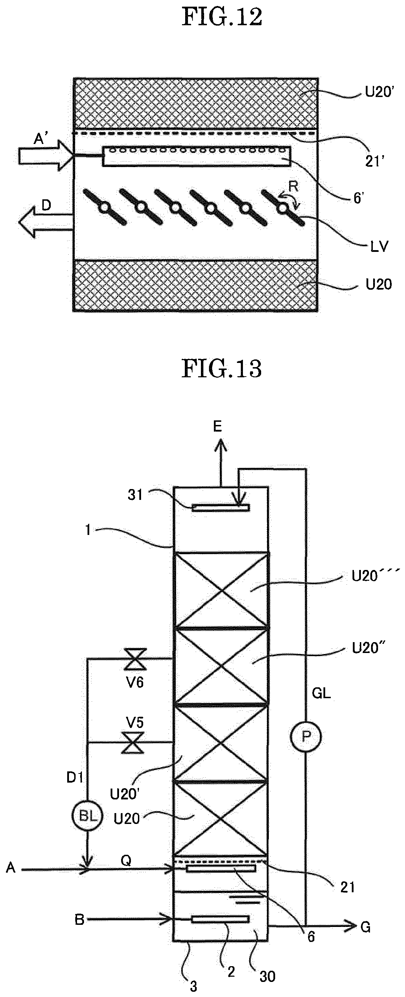

The fixed bed is filled in with the carrier and fixed and is made of, for example, a reticular frame into which a great number of pieces of the carrier is put, which is placed on a support net 21 that is provided in a predetermined place in the reactor tank. It is also possible, as shown in FIGS. 10 and 13, to form the fixed bed of a plurality of separated fixed bed units (U20, U20', ...).

The higher the filling factor of the carrier is relative to the bioreactor tank, the greater the amount of carrier per volume of the bioreactor tank is, the greater the number of microorganisms that are attached and fixed to the carrier can be held, and the higher the gained treatment performance is. Meanwhile, the filling factor of the carrier is set to 60 V% to 80 V%, preferably 50 V% to 70 V%, taking into consideration that the gas flow should be uniform within the bioreactor tank and the gas should be mixed in for the gas phase.

As for the shape of the anaerobic bioreactor tank, either rectangular or cylindrical is possible. It is more desirable to be cylindrical, taking into consideration that the filler carrier and the supplied gas flow should be uniform. In the case of a cylindrical tank, the carrier can be filled in approximately uniformly, which also makes the flow of the supplied gas uniform throughout the entire body.

A liquid that contains a nutrition source for the organisms is supplied to the fixed bed 20. It is possible to constantly or intermittently supply the liquid from the liquid storage unit 3 through the liquid circulating line GL by means of a liquid circulating pump P. The liquid is sprinkled from the upper portion of the fixed bed 20 by way of a liquid supplying means 31 such as an ejection pipe so as to make uniform contact with the carrier layer with which the inside of the fixed bed is filled in. As a result, the nutrition source in the liquid is supplied to the microorganisms on the surface of the carrier (biofilm that covers the surface of the carrier) in order to maintain the high activity of the microorganisms, which allows a stable methane generating rate to be achieved.

Here, at an early stage of the starting up of the device, where a sufficient number of microorganisms has not yet attached to the surface of the filler carrier, it is necessary to put, as seed sludge, the digested sludge from methane generating bacteria containing sewage or the digested sludge gained through methane fermentation treatment of organic waste into the liquid storage unit 3 in advance in order to allow microorganisms, which are mainly methane generating bacteria, to attach to the surface of the carrier.

The greater the amount of seed sludge that is put into the liquid storage unit 3 is at the time of starting up, the greater the amount of sludge that attaches to the surface of the carrier is, which makes starting up possible in a shorter period of time. Meanwhile, in the case where the amount of seed sludge that is put in is excessive, a large amount of sludge accumulates not only on the surface of the carrier, but also in the gaps between the pieces of the filler carrier, which locally blocks the gaps and prevents the supplied hydrogen and carbon dioxide gases from flowing uniformly and from making contact with the surface of the carrier, and thus, a stable treatment performance cannot be achieved.

It is necessary to determine the amount of seed sludge to be added at the time of starting up, taking into consideration the amount of filler carrier, the shape of the carrier and the filling factor. In general, the seed sludge that is put in per liter of the amount of the filler carrier is 0.5 g to 5 g, preferably 0.5 g to 2.5 g.

The SS concentration of the liquid into which the seed sludge is put at the time of starting up is set to 2,500 mg/L to 15,000 mg/L, preferably 2,500 mg/L to 10,000 mg/L. When the SS concentration (the mass concentration of the floating substance) is high, sludge accumulates in the filler carrier layer, thereby increasing the risk of blocking the flow path of the liquid or the supplied gas. Therefore, it is preferable to dilute the liquid with treated sewage or the like in the case where the SS concentration of the digested sludge that becomes the seed sludge is high.

As for the circulation of the liquid, it is preferable to set the flow amount of the circulated liquid by taking into consideration the volume of the filler carrier layer. Usually, it is necessary for the flow amount of the circulated liquid per day to be 2 to 20 times greater, preferably 5 to 10 times greater, than the volume of the filler carrier.

When the flow amount of the circulated liquid is small, the contact between the liquid and the microorganisms that are attached to the surface of the filler carrier is not uniform, and therefore, the nutrition source in the liquid cannot be stably supplied to the microorganisms, which lowers the activity of the microorganisms and prevents the methane generating rate from being stable. Therefore, it is important to maintain an appropriate flow amount of the circulated liquid.

This liquid includes a nutrition source for the microorganisms (methane generating bacteria) as shown in Table 1.

**[Table 1]**

| Added Nutrient Elements and Concentrations | | | |
|---|---|---|---|
| Item | Element | Optimal concentration (mg/L) | Range of neccesary concentration (mg/L) |
| Nutrient salt | N | 50 | 10 ~ 100 |
| | P | 10 | 2 ~ 20 |
| | S | 5 | 1 ~ 10 |
| Trace element | Fe | 10 | 2 ~ 20 |
| | Al | 2 | 0.4 ~ 4 |
| | Co | 0.02 | 0.01 ~ 0.1 |
| | Ni | 0.02 | 0.01 ~ 0.1 |
| | Zn | 0.02 | 0.01 ~ 0.1 |
| | Cu | 0.02 | 0.01 ~ 0.1 |
| | Mn | 0.02 | 0.01 ~ 0.1 |
| | Mo | 0.05 | 0.01 ~ 0.1 |
| | Se | 0.05 | 0.01 ~ 0.1 |
| | W | 0.02 | 0.01 ~ 0.1 |
| | B | 0.02 | 0.01 ~ 0.1 |
| Cation | Na⁺ | 100 | 50 ~ 500 |
| | K⁺ | 200 | 100 ∼ 400 |
| | Ca²⁺ | 100 | 50 ~ 100 |
| | Mg²⁺ | 75 | 25 ~ 250 |

The device for generating methane gas that involves the removal of carbon dioxide according to the present invention is characterized as shown in FIG. 3 in that a portion of the treated gas E that has been discharged from the anaerobic bioreactor tank 1 is circulated (symbol D) to the reactor tank 1. As for the circulation, it is possible to force the circulation by using a gas blower BL. In addition, it is not only possible for the circulated treated gas to be mixed with the gas A and/or the gas B before being supplied to the air diffusing means 2, but also for a gas diffusing means to be provided exclusively for the circulated gas.

As shown in FIGS. 8 and onwards, in the case where the gas A and the gas B are separately injected, concretely speaking, the gas A that contains CO₂ is introduced into the gas phase that is filled in with the carrier, in particular, the space provided in the lower portion of the fixed bed, whereas the gas B that contains hydrogen is introduced into the liquid storage unit. This is done in order to prevent the methane generating reaction from stagnating when a large amount of carbon dioxide dissolves in the liquid, causing a CO₂ deficit that is to be supplied to the microorganisms that are attached to the carrier. Furthermore, this is done in order to prevent the pH of the circulated liquid from lowering or impurities from being generated due to the dissolving of CO₂.

In the case where the gas A and the gas B are separated as shown in FIGS. 8 and onwards, it is preferable for the circulated gas, which is a portion of the treated gas that is circulated, to be injected into the same gas phase as the gas A because it includes untreated carbon dioxide. As shown in FIGS. 15, 17, 18, and 19, in the case where a plurality of reactor tanks is connected in order to carry out treatment, it is possible to inject a circulated gas D2 into the liquid in order to stir the liquid 30' that accumulates in the liquid storage unit 3' in the reactor tank 1' on the downstream side (at a later stage).

The purpose of circulating a portion of treated gas is effectively use hydrogen and carbon dioxide that stay in the treated gas without having been treated so that the methane gas conversion efficiency can be made close to 100 %. The circulation of the treated gas increases the frequency of contact between the microorganisms and carbon dioxide and hydrogen gases that are reused in the anaerobic bioreactor tank, which increases the methane gas conversion efficiency. As described above, an increase in the amount of generated mist from the liquid 30 and an acceleration of the dissolving of the hydrogen and carbon dioxide gases into the liquid can also be expected. Furthermore, hydrogen that is light tends to concentrate in the upper portion of the gas phase within the reactor tank 1. An increase in the amount of circulated gas makes the gas flow in the gas phase stronger, which makes the hydrogen gas, though it is light, uniform within the gas phase. As a result, the frequency of contact of either the hydrogen gas or the carbon dioxide gas with the microorganisms increases, and thus, a stable treatment performance and treatment rate can be gained.

As described below, even in the case where the circulation of the treated gas fluctuates the amount of supply of the gas A or B or fluctuates the CO₂ concentration in the supplied gas A or the hydrogen concentration in the gas B, it becomes possible to carry out bacterial treatment stably.

FIG. 4 is a diagram where various types of apparatuses (supply pumps, control valves, concentration sensors, and the like) are arranged in the methane gas generating device in FIG. 3 in order to increase the methane gas conversion efficiency. The gas A is supplied by means of a supply pump P1, and the gas B is supplied by means of a supply pump P2. In FIG. 4, the gas A and the gas B are mixed so as to be supplied to the anaerobic bioreactor tank 1 together with the treated gas that is to further be circulated; however, as shown in FIGS. 8 and onwards, needless to say, it is possible to supply the respective gases to the reactor tank 1 separately. In addition, in the case where a high-pressure gas is supplied from the supply source for the gas A or B, it is possible to form the system with a supply amount adjusting valve (control valve) in place of the supply pump. In the case where the inner pressure of the treated gas to be circulated is high, it is possible to arrange a check valve along the line on the supply side that is connected to the gas circulating line in order to prevent a portion of the treated gas from flowing oppositely towards the supply side of the gas A or B.

Furthermore, it is also possible to arrange a concentration sensor S1 for measuring the CO₂ concentration and the hydrogen concentration of the gas that is supplied to the reactor tank 1, or a concentration sensor S2 for measuring the concentration of methane gas that is included in the treated gas. It is also possible to drive or control supply pumps P1 and P2, an air blower for circulation BL, a control valve V2, and the like by means of a control means CU such as a computer on the basis of the measurement results of these various types of sensors. Dotted-line arrows in the figures indicate communication routes of the electric signals. Here, a dotted-line arrow is not shown for the control valve V2 and the like; however, it is naturally possible to form these components in such a manner that they are controlled by the control means CU.

An example of treatment by means of the control means CU is described below when the target of control by means of the control means CU is set to a case where all of the supply amounts of CO₂ that is included in gas A and hydrogen that is included in gas B are treated by the microorganisms within the anaerobic bioreactor tank 1, that is to say, the conversion efficiency is 100 %.

When a CO₂ flow amount a (L/h) and a hydrogen flow amount b (L/h) are assumed as the supply amounts per unit hour (supply flow amount), the hydrogen flow amount b becomes b = 4a with the carbon dioxide gas flow amount a being the standard, and the generated methane gas flow amount e (L/h) becomes e = a in order for the reaction in the reaction formula (1) to be achieved.

In order for the above to be achieved, not only the supply amounts of the gas A that contains carbon dioxide and the gas B that contains hydrogen are adjusted so that the supply amounts of carbon dioxide and hydrogen that are supplied to the reactor tank 1 can be adjusted, but also the molar ratio of carbon dioxide to hydrogen in the supplied gas is adjusted to 1:4, for example. Furthermore, it is possible for the supply amount of hydrogen gas to be made greater than the above-described molar ratio, taking into consideration the fact that carbon dioxide dissolves in the liquid more easily than hydrogen, for example. In addition, this molar ratio can be expressed as the mass ratio in the supplied gas in such a manner that the mass ratio of hydrogen to carbon dioxide in the supplied gas is set within a range from 1:5 to 1:11.

Meanwhile, in the case where the gas A and the gas B are injected separately as shown in FIGS. 8 and onwards, the CO₂ containing gas A is directly injected into the gas phase that is filled in with a carrier, which almost completely prevents the gas A from dissolving in the circulated liquid. It is preferable for the mass ratio of hydrogen to carbon dioxide in the supplied gas to be 1:5 to 1:8, taking into consideration the facts where H₂ that has been injected into the liquid storage unit barely dissolves in the circulated liquid, and instead, a portion thereof is used for the synthesis of bacterial cells of the microorganisms.

As for the supply amounts of the gas A and the gas B, the concentration sensor S1 measures the concentrations of carbon dioxide gas and hydrogen, and the control means CU controls the driving of the respective supply pumps P1 and P2 so that the concentration ratio or the mass ratio that is calculated from the respective concentrations becomes a predetermined value.

The concentration sensor S2 for measuring the concentration of methane gas is provided in order to determine whether or not the supplied carbon dioxide or hydrogen are sufficiently treated by the microorganisms within the reactor tank 1. In addition, it is possible for the concentration sensor S2 to detect the concentrations of carbon dioxide and hydrogen that are included in the remaining gas that has not been treated in order to evaluate the treatment performance of the microorganisms within the reactor tank 1. In the case where the amount of the supplied material gas exceeds the treatment performance of the microorganisms, the control means CU can control the supply amounts of the gas A and the gas B.

The circulated amount of the circulated gas D is set on the basis of the CH₄ concentration in the treated gas E and the flow state thereof and the like within the reactor tank 1. In order to control the circulated amount, it is possible to control not only the driving of the air blower BL, but also the opening and closing of the control valves V2 or V3 or the amount by which these valves are opened and closed, if necessary.

As described above, the supply amounts of gases A and B, which are supplied gases, can be controlled in order to make it possible to convert the entirety of the carbon dioxide (and hydrogen) in the supplied gas to methane gas by means of the microorganisms within the reactor tank 1. In the case where the supply amount of the gas A or B fluctuates or the CO₂ concentration in the supplied gas A or the hydrogen concentration in the gas B fluctuates, however, it is difficult to control the supply amounts of gases A and B optimally with high precision. In addition, there are some cases where the total amount of microorganisms fluctuates due to the operation time, the temperature of the environment, and the like, which makes the control of the supply amounts of gases A and B more difficult.

In contrast, when the circulated gas D is provided, the amount of the gas that is supplied to the reactor tank 1 (including the circulated gas) can be increased relative to the supply amounts of gases A and B (the supply amounts of the supplied carbon dioxide and hydrogen) so that the effects of the fluctuation factors of the gases A and B can be mitigated, and thus, stable microbial treatment can be achieved. The higher the ratio (magnification) of the circulated amount of circulated gas to the supply amounts of gases A and B is, the more stable the operation becomes, and in addition, the better the flow state becomes within the reactor tank 1, which can be said to be preferable. Furthermore, the gas circulation magnification at the time of stable treatment is set to approximately 10 times, preferably 2 to 20 times, taking into consideration the power consumption by the air blower BL and the gas HRT (the time during which the material gas stays in the reaction liquid). Here, the magnification of the circulated gas amount is the magnification relative to the total amount of the injected CO₂ gas and the H₂ gas.

In the case where the concentration sensor S2 collects and measures a sample gas where the CH₄ concentration is as low as 60 % or less, for example, it is possible to temporarily increase the circulated gas amount to deal with the situation. At an early stage of starting up, or the like, where the concentration of microorganisms is low within the anaerobic bioreactor tank 1 with insufficient activity, the total amount of the supplied gas A and the gas B is small, and therefore, it is preferable for the circulated gas magnification to be 10 times or greater than the supplied gas amount with 50 times greater being the maximum.

Oxygen cannot be supplied to the microorganisms, and therefore, the risk of oxygen being mixed in can be suppressed when the amount of circulated gas is increased, rather than when the amounts of gases A and B are increased.

As for a higher efficiency of conversion into methane gas in the anaerobic bioreactor tank 1, the conversion efficiency can be increased by constantly measuring the methane gas concentration in the treated gas within the anaerobic bioreactor tank 1 while adjusting the supply flow amounts of the supplied gas A that includes carbon dioxide gas and the gas B that includes hydrogen gas.

The respective supply pumps P1 and P2 are controlled by using the concentration sensor S1 in order for the respective flow amounts of gases A and B to be adjusted so that the mass ratio of H₂:CO₂ becomes 1:5 to 1:11, for example. When the mass ratio of H₂:CO₂ between the gases that are supplied to the anaerobic bioreactor tank 1 becomes a value out of the range from 1:5 to 1:11, the supply amount of H₂ or CO₂ exceeds the necessary amount thereof for the methane generating reaction as the methane generating reaction proceeds, and therefore, a large amount of H₂ or CO₂ remains after the methane generating reaction, which causes the CH₄ concentration to lower in the treated gas. In addition, the discharged gas amount increases, which makes the separation load applied to the below-described gas separating device become excessive, thereby making the CH₄ separating performance of the gas separating film unstable.

As for the method for dealing with the case where the CH₄ concentration in the treated gas is detected to be as low as 50 % or lower by the concentration sensor S2, the flow amounts of gas A and gas B are adjusted so that the H₂:CO₂ mass ratio in the supplied gas becomes 1:5 to 1:11. In the case where the CH₄ concentration is as low as 50 % or lower, even when the H₂:CO₂ mass ratio in the supplied gas is already within the range from 1:5 to 1:11, it becomes possible to increase the CH₄ concentration in the treated gas by adjusting the H₂:CO₂ mass ratio in the supplied gas to 1:5 to 1:7, preferably 1:5 to 1:6.

In the case where the carbon dioxide concentration can be detected by the concentration sensor S2, the supply amount of gas A is increased to such a degree that carbon dioxide can be detected, and in addition, the supply amount of gas B is adjusted so that the H₂:CO₂ mass ratio in the supplied gas becomes within a range from 1:5 to 1:11. Naturally, it is also possible to change the mass ratio to be in a range from 1:5 to 1:7 or from 1:5 to 1:6 so that the conversion efficiency of methane gas can be increased and maintained as high as 85 % or higher, for example.

As the methane generating reaction progresses, a small amount of H₂O is generated as a byproduct, and the level of the liquid in the liquid storage unit 3 increases within the reactor tank 1. In order to make the level of the liquid 30 constant, it is necessary to discharge the liquid from somewhere near the lower portion of the reactor tank 1 by the amount that has been increased as methane gas is generated. As for the method for discharging the liquid G, the valve V1 is controlled to discharge a predetermined amount of liquid G when the level of the liquid storage unit 3 exceeds a predetermined level. It is also possible to maintain the level of the liquid storage unit 3 at approximately constant by continually discharging a predetermined amount.

In the case where floating microorganisms exist at a high concentration in the discharged liquid, solids and liquids can be separated to feedback only the microorganisms to the liquid storage unit 3 so that the concentration of microorganisms within the reactor tank can be maintained high, and thus, a stable methane generating performance can be achieved. As for the solid-liquid separation means, it is possible to use a membrane separation or a centrifugation separation system. In this manner, solid-liquid separation is carried out on the discharged liquid if necessary while the sludge concentration within the liquid storage unit is being confirmed, and thus, it is possible to return the microorganisms to the liquid storage unit 3. Here, in the case where the sludge amount that adheres to the carrier increases as the microorganisms proliferate and the sludge concentration of the circulated liquid increases as the biofilm peels to such a high concentration as to affect the circulation, it is desirable to discharge the sludge from within the tank appropriately.

As the liquid G is discharged, the nutrition that is necessary for the microorganisms is also discharged to the outside of the bioreactor tank together with the discharged liquid, and therefore, it is necessary to replenish the discharged nutrition. Such replenishment may be carried out together with the replenishment F of the liquid or only the nutrient elements that have run short may be injected. Examples of the nutrient elements to be added and the concentrations thereof are shown in Table 1. As shown in Table 1, the types of nutrients can be basically divided into nutrient salts, trace elements, and cations. The concentrations shown in Table 1 are concentrations in the liquid within the anaerobic bioreactor tank. Therefore, it is preferable to add the amounts of nutrient elements in accordance with the amount of the discharged liquid that accompanies the water generation in the methane generating reaction (the amounts of nutrient elements that are discharged together with the discharged liquid) so that the concentrations in the liquid can be maintained within the ranges shown in Table 1. In particular, it has been confirmed that the residual concentration in the reaction liquid lowers as NH₄-N gradually vaporizes as NH₃ through ventilation, and therefore, it is necessary to periodically add an NH₄-N source. As for the addition of a nutrient source (replenishment F), the same effects can be gained for both continuous or batch processes.

As the methane generating reaction proceeds, H₂O is generated, and an increase in the circulated liquid amount lowers the M-alkali level of the circulated liquid. When the M-alkali level is lower than 100 mg/L, the pH is also lowered to indicate weak acidity. In order for a stable methane generating reaction to be gained, it becomes necessary to continuously add an alkali chemical to the circulated liquid so that the M-alkali level becomes 100 mg/L or higher, preferably 200 mg/L or higher.

The same effects can be gained when using any alkali chemical from among sodium hydroxide, sodium carbonate, sodium bicarbonate, calcium hydroxide, and magnesium hydroxide.

The amount of the alkali chemical to be added depends on the H₂ injection load, and usually, the necessary amount of alkali chemical to be injected is 5 to 50 mg/g-H₂ per mass of consumed H₂. That is to say, the M-alkali level that corresponds to 0.5 % to 5 % of the mass of consumed H₂ needs to be injected as an alkali chemical.

When the injection amount of an alkali chemical is greater than the necessary amount, the pH of the circulated liquid may become high, and therefore, it is preferable to use a sodium bicarbonate solution that causes a slight change in the pH.

In addition, in order to make the change in the pH due to the injection of an alkali chemical slight, it is possible to control the alkali chemical injection so that the pH becomes in a predetermined range while continuously monitoring the pH of the circulated liquid. For example, it is possible to control an alkali chemical injection pump in such a manner that the pump is operated when the pH of the circulated liquid becomes 6.0 or lower, and the alkali chemical injection is stopped when the pH increases to 8.0. Furthermore, it is possible to change the pH range to monitor the alkali chemical injection if necessary. It is preferable for the pH to be 6.5 to 7.5 in the case where the fluctuation in the flow load is small. A continuous injection of a predetermined amount is also possible when the treatment is stable.

As for the addition of a nutrient source or an alkali chemical to the circulated liquid, the subject material may be injected into the liquid 30 in the liquid storage unit 3 as indicated by arrow F in FIG. 3, or may be injected into the circulated liquid in the middle of the liquid circulating line GL (dotted-line arrows F1 or F2). In the case where the injection point is located as indicated by F1 along the circulated liquid line GL, the mixing of the added material with the circulated liquid is accelerated by the liquid circulating pump P, and thus, the material is mixed uniformly in the circulated liquid with pH fluctuation in the circulated liquid being low, and thus, a stable treatment performance can be achieved.

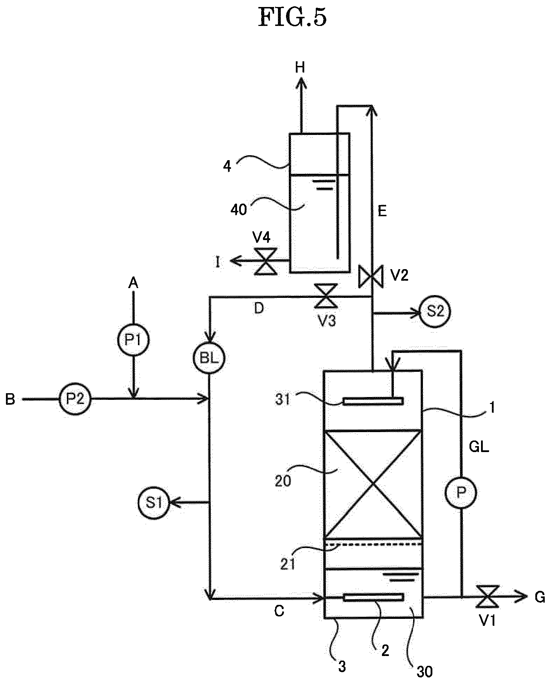

FIG. 5 shows another example of the present invention where an NH₃ removing device, which is an NH₃ removing means, is added at a later stage of the bioreactor tank. Here, the treated gas E is introduced from the anaerobic bioreactor tank 1 into the NH₃ removing device 4. Clear water or a weak acidic water solution 40 is placed in the NH₃ removing device 4, where a vaporized NH₃ gas is dissolved in the water solution so as to gain NH₄-N. In the case where NH₃ has dissolved in water, the solution becomes slightly alkaline as indicated in the reaction formula NH₃ + H₂O → NH₄OH. Therefore, it is desirable for the water solution in the tank to be weakly acidic in advance.

The solution in the NH₃ removing device that has absorbed NH₃ as a result of dissolving is periodically discharged (I) through a drain for NH₃ absorbed liquid. The discharged liquid I can be reused as an N nutrient source for the reaction liquid.

The end of the pipe through which the treated gas E is discharged from the anaerobic bioreactor tank is introduced to the bottom portion of the NH₃ removing tank, and therefore, the depth of the water in the NH₃ removing tank corresponds to the water head pressure applied to the anaerobic bioreactor tank. The greater the water depth of the NH₃ removing tank is, the higher the water head pressure applied to the inside of the anaerobic bioreactor tank becomes. The higher the water head pressure is, the higher the inner pressure of the anaerobic bioreactor tank is, where a great amount of hydrogen gas that is difficult to be dissolved in the reaction liquid can be made to dissolve, the methane generating reaction by microorganisms can be accelerated, and thus, a high methane generating rate and a high conversion efficiency to methane gas can be expected.

As for the shape of the container of the NH₃ removing device into which a water solution or the like is placed, a column is preferable due to the smaller area for installation. NH₃ is at a low concentration when conventionally vaporized and the solubility of NH₃ in water is high, and therefore, a necessary amount of water in the removing device may be small. As a result, a long column is more preferable. It is preferable for the water depth to be 3 m to 10 m, which is approximately the same as the water depth of the reactor tank 1.

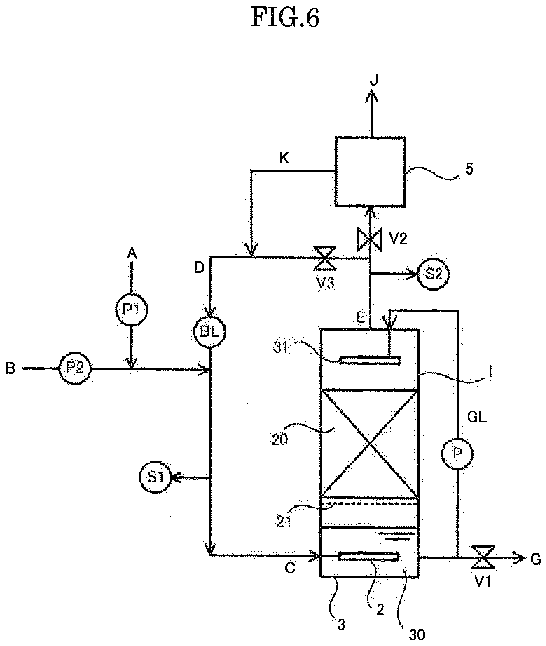

FIG. 6 shows another example of the present invention where a gas separating device 5, which is a gas separating means, is added at a later stage of the anaerobic bioreactor tank. In order to gain a highly pure methane gas by purifying the treated gas from the anaerobic bioreactor tank 1, the treated gas E is introduced into the gas separating device so that methane gas is separated and purified, and thus, CH₄ gas having high purity is gained as the treated gas L. Meanwhile, the mixed gas of the remaining hydrogen and carbon dioxide is merged with the circulated gas D in the anaerobic bioreactor tank through the reconveyance line for the separated residual gas N after the separation and purification of methane gas. The merged gas is supplied to the air diffusing means 2 in the bottom portion of the anaerobic bioreactor tank 1 so as to be reused in the methane generating reaction.

Here, the residual gas separated from the gas separating device can be reconveyed directly to the bioreactor tank so that the residual H₂ and CO₂ can be used in the methane generating reaction in the same manner through the mixing and stirring of the circulated gas inside the bioreactor tank, and thus, the same effects can be gained.

As the CH₄ separating system in the gas separating device 5, a gas separation membrane method, a pressure swing adsorption method (PSA method), or a cryogenic separation method can be used. From among these, a membrane separating type separating device, where CH₄ gas is condensed and CO₂ and H₂ gases pass through is compact and simple, has high efficiency and is easy to operate, and therefore, it is preferable to adopt this device.

As for the treatment conditions of the gas separating membrane, it is appropriate for the pressure on the gas supplying side to be 50 kPa to 500 kPa. The higher the pressure on the supplying side is, the higher the gained gas separation rate and separating performances are; however, it is preferable for the pressure on the supplying side to be 50 kPa to 250 kPa, taking the membrane contamination, the necessary power, and the like into consideration. It is also possible to provide a pressure pump to the inside of the gas separating device or the pipe that is connected to the gas separating device.

In the case where the gas that is supplied to the gas separating device contains a poisonous component for the gas separating membrane, the CH₄ separating and purifying performances deteriorate, which causes the lowering of the gas permeation rate. General examples of such poisonous components of the gas separating membrane include siloxane or hydrogen sulfide in the biogas. Therefore, it is desirable to separately provide a treating device for removing the poisonous components in advance from the treated gas from the anaerobic bioreactor tank 1.

As for the gas separating membrane, it is desirable for a gas separating membrane in one stage to separate and purify CH₄ gas; however, gas separating membranes in two stages may be provided depending on the separation properties of the gas separating membrane and the components or the properties of the supplied gas. For example, it is possible to separate CO₂ gas at the first stage, and after that, to separate H₂ gas at the subsequent second stage.

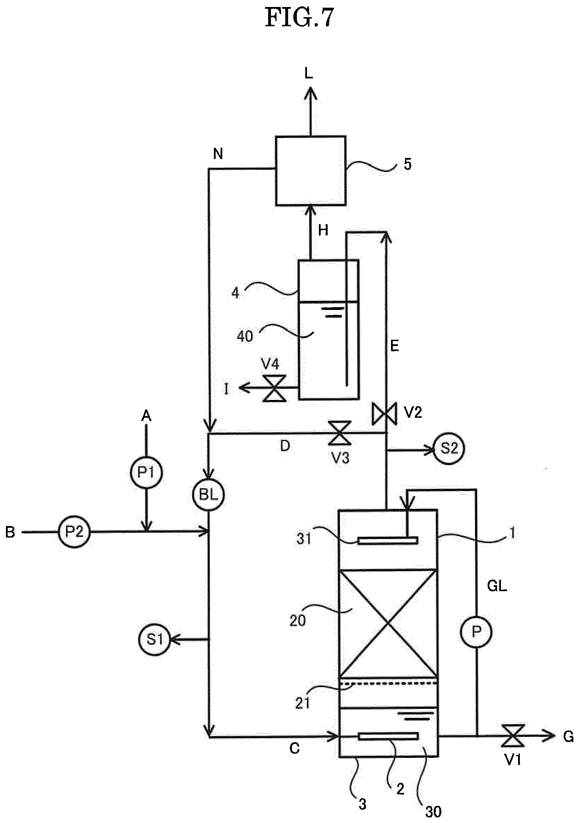

FIG. 7 shows another example of the present invention where an NH₃ removing device 4 and a gas separating device 5 are connected to the anaerobic bioreactor tank 1.

Here, the treated gas H from the NH₃ removing device 4 is introduced into the gas separating device 5. The treated gas L from the gas separating device 5 is methane gas having high purity, whereas hydrogen and carbon dioxide containing gases that remain after the separation are introduced into the circulated gas in the anaerobic bioreactor tank as separated remaining gas N.

An example where gas A that is CO₂ containing gas and gas B that is hydrogen containing gas are separately introduced into the anaerobic bioreactor tank 1 is described in detail in reference to FIGS. 8 and onwards. Here, the injections of a nutrient liquid and an alkali liquid as shown in FIG. 3, the various types of controls using various types of sensors in FIG. 4, the removal of ammonia in FIG. 5, and the separation of gas in FIG. 6 are not particularly described in reference to FIGS. 8 and onwards; however, needless to say, they can be applied in the same manner as in the embodiments in FIGS. 8 and onwards. At the time of such applications, the structures of the reactor tank 1 and the gas circulating line D and the liquid circulating line GL that surround the reactor tank 1 may be adjusted. It is also possible to individually incorporate various types of sensors into the line for gas A, the line for gas B, and the like if necessary.

In FIG. 8, a fixed bed 20 is provided in a reactor tank 1, and gas A is supplied to the space (gas phase) between the lower portion of the fixed bed 20 and a liquid storage unit 3. As a result, carbon dioxide gas can be suppressed from dissolving in the circulated liquid. Meanwhile, gas B is supplied to the liquid 30 in the liquid storage unit 3, which contributes to the stirring of the liquid.

An air diffusing means 6 is used for gas A and is arranged in the vicinity of the lower surface of the fixed bed 20, and thus, is formed so that carbon dioxide can be supplied efficiently to the fixed bed. Meanwhile, hydrogen contained in gas B is not dissolved much in the liquid 30, and most of it ends up passing through the fixed bed.

FIG. 9 shows the methane gas treating device in FIG. 8 that is provided with a gas circulating line D. The circulated gas includes untreated carbon dioxide gas, and therefore is supplied to the gas phase in the same manner as gas A. Gas A and circulated gas D may be merged before being supplied to the gas phase as shown in FIG. 9, or may be supplied separately.

FIG. 10 shows two separate fixed bed units (U20, U20') instead of the fixed bed 20 in FIG. 8. A gap is created between the adjacent fixed bed units, and gas A' is introduced into this gap. Even in the case where gas A' is introduced, the introduction of gas A in the lower portion of the fixed bed unit 20 cannot be omitted. Here, the gas that contains carbon dioxide is divided into gas A and gas A', which makes it possible to adjust the ratio of division and the gas amounts supplied to the lower portions of the respective fixed bed units while observing the treatment state in the fixed bed unit U20 and the other fixed bed unit U20'.

As for the method for evaluating the treatment performance of each fixed bed unit, the supply amount or the division ratio of the supplied gas A or A' can be varied, and a gas detection sensor is arranged in the vicinity of each fixed bed unit in the upper portion of the unit, and thus, a method for detecting the methane gas concentration or the residual hydrogen concentration can be provided, or the treatment performance of each fixed bed unit can be evaluated from the change in the concentration of methane gas that is included in the treated gas E discharged from the reactor tank 1.

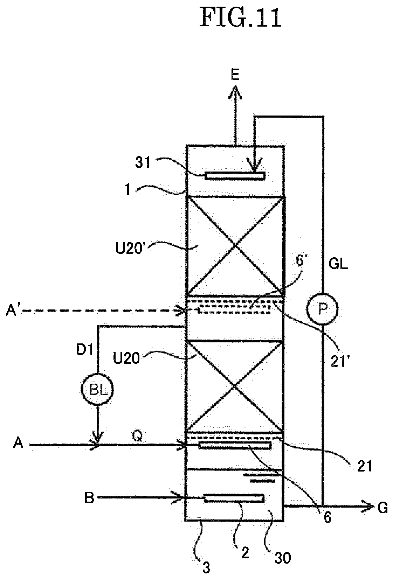

FIG. 11 shows an embodiment where a gas circulating line is provided in the methane gas treating device in FIG. 10. The present embodiment is characterized in that a portion of the treated gas is sampled from the space between the fixed bed units (between U20 and U20') as circulated gas (see symbol D1) so as to be circulated on the upstream side of the fixed bed. There is a possibility of the circulated gas containing a large amount of carbon dioxide gas, and therefore, the circulated gas is returned to the gas phase in the lower portion of the fixed bed in the same manner as gas A. Naturally, the circulated gas may be merged with gas A or separately returned.

In the case where gas A' is introduced into the space between the fixed bed units in FIG. 11 in the same manner as in FIG. 10, it is required to devise the system in such a manner that, as much as possible, the introduced gas A' and the treated gas that is led out as the circulated gas are not mixed. For example, as shown in FIG. 12, a plurality of louvres LV is arranged in the space between the fixed bed units so as to control the direction in which the treated gas flows, and at the same time, an air diffusing means (air diffusing pipe 6') having a great number of air diffusing pores is arranged on the upper side of the louvres LV. This configuration allows a portion of the treated gas that has been treated in the fixed bed unit U20 in the lower portion (upstream side in the direction in which the gas flows) to be led out as the circulated gas D, whereas the gas that has been introduced as gas A' is efficiently supplied to the next fixed bed unit (U20'). Naturally, the treated gas amount that flows upward (on the downstream side of the flow of the gas) can be adjusted by rotating R the plurality of louvres LV in the same direction. The degree of opening or closing can, of course, be adjusted with the louvres, and the flow of the circulated liquid that flows downward from the upper portion cannot be prevented.

FIG. 13 is a diagram showing an example where the circulated gas is directly sampled from the middle of the fixed bed units that are formed in two or more stages. The fixed bed units have a sufficient space for the gas to move inside, and a pipe can be introduced in this fixed bed in such a manner that a gas that moves through the fixed bed units can be easily sampled through suction by means of the air blower BL. In FIG. 13, the circulated gas is selectively sampled from some of the four fixed bed units (U20, U20', U20", U20‴) by using control valves (V5, V6) during the treatment. Needless to say, it is possible for the system to be formed of only one fixed bed unit instead of the four fixed bed units as shown in FIG. 13. As described above, the CH₄ generating reaction is made to proceed by a certain degree by means of the circulated gas in lower fixed bed units in the middle of the treatment, and the finishing treatment is carried out in upper fixed bed units, and thus, it becomes possible to gain CH₄ gas at a high concentration.

FIG. 14 shows an embodiment where the two fixed bed units (U20, U20') shown in FIG. 10 are incorporated into separate treatment tanks (1, 1'). The treated gas E1 that has been treated in a reactor tank 1 is supplied to the gas phase in a lower portion of the fixed bed unit U20' in the other reactor tank 1'. In addition, the circulated liquid that has been supplied to the treatment tank 1' is supplied to the fixed bed unit U20' through the ejection pipe 31' so as to be stored in the liquid storage unit 3' as a liquid 30'. The liquid 30' is supplied to the reactor tank 1 through a liquid circulating line GL1.

The liquid 30 that has been stored in the liquid storage unit 3 in the reactor tank 1 is to be resupplied to the reactor tank 1' through the liquid circulating line GL.

FIG. 15 shows the configuration where a gas circulating line D2 is provided only in the reactor tank 1' on the downstream side (rear stage) in the device for generating methane gas in FIG. 14 so that the liquid 30' that has been stored in the liquid storage unit 3' within the reactor tank 1' can be stirred. At the time of stirring, the treated gas is released from an air diffusing means 2'. The treated gas that has been treated in the reactor tank on the downstream side contains almost no CO₂ gas, and therefore, the content of CO₂ gas is so low that the amount of CO₂ gas that dissolves in the liquid 30' can be ignored even when the treated gas is used to stir the liquid 30', and the possibility of the above-described problem, such as the generation of impurities or the like, is very low.

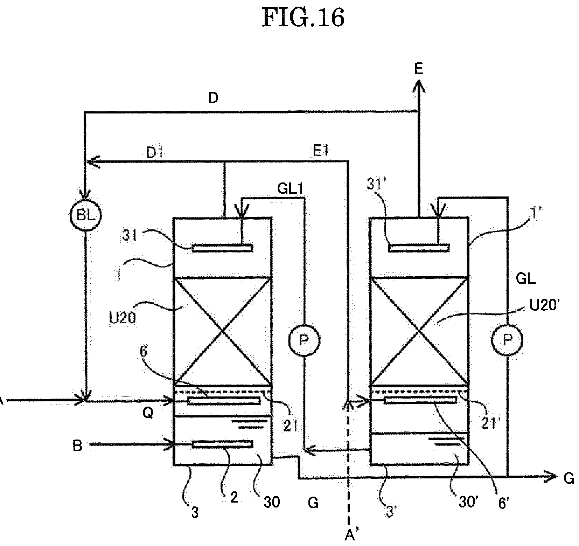

FIG. 16 shows the configuration where a methane gas treating device using a plurality of reactor tanks (1, 1') as shown in FIG. 14 is provided with gas circulating lines. It is possible to provide a gas circulating line D for returning the treated gas E that has been discharged from the second reactor tank 1' to the upstream side of the entire treatment system, and it is also possible in the same manner as in FIG. 11 to return a portion of the treated gas that moves between adjacent fixed bed units (U20, U20') to the upstream side of the fixed bed unit U20 as circulated gas D1. The gas circulating lines D and D1 may coexist in the system, or only one of them may be provided. Here, the above-described "circulated amount of treated gas" means the total circulated gas amount, which is the sum of the circulated gases D and D1.

In the case where gas A' is supplied in either system in FIG. 14 or FIG. 16, it is possible to mix the gas A' with the treated gas E1 from the reactor tank 1, or it is also possible to directly introduce the gas A' into the gas phase in the lower portion of the fixed bed unit U20' within the reactor tank 1'.

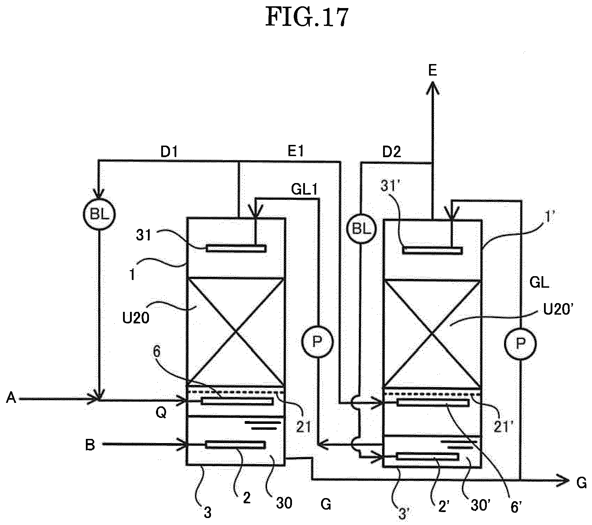

FIG. 17 shows an example of the application of the methane gas generating device shown in FIG. 16 where the configuration allows a portion of the treated gas in the reactor tank 1' to be supplied to the air diffusing means 2' in the liquid storage unit 3' by using a gas circulating line D2 so as to stir the liquid 30' in the same manner as in the embodiment in FIG. 15. Here, a new gas circulating line D2 is installed instead of the gas circulating line D as shown in FIG. 16. As for the "circulated amount of a treated gas" in the methane gas generating device in FIG. 17, the portion of the circulated amount at a previous stage is that of the circulated gas D1, and the portion of the circulated amount at a later stage is that of the circulated gas D2.

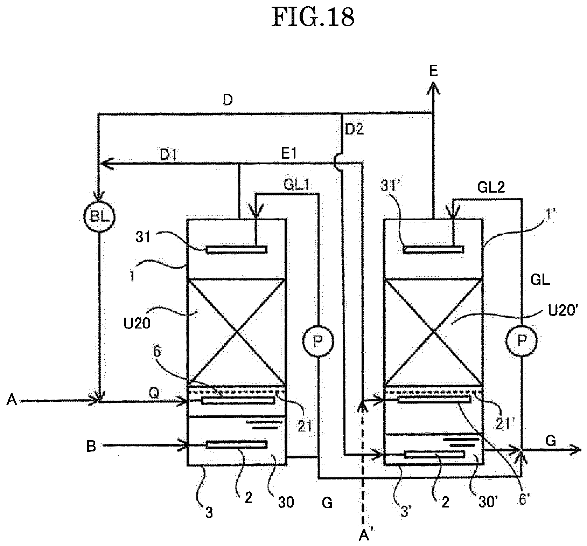

FIG. 18 shows the configuration where the flows of the circulated gas and the circulated liquid are provided for each reactor tank (1, 1') so that the state of the fixed bed unit in each reactor tank can be adjusted optimally. Concretely, the configuration of the reactor tank 1 allows the gas circulating line D1 to circulate the treated gas that has been treated in the reactor tank 1. As for the circulated liquid, the configuration also allows the liquid circulating line GL1 to circulate the circulated liquid that has been treated in the reactor tank 1.

As for the reactor tank 1', the configuration allows the gas circulating line D2 to circulate the gas that has been treated in the reactor tank 1' so that the treated gas is again led into the reactor tank 1'. As for the circulated liquid, the configuration also allows the liquid circulating line GL2 to circulate the circulated liquid to be used in the reactor tank 1' only through the reactor tank 1'. Further functions are added in such a manner that it is made possible for the circulated liquid from the reactor tank 1 to be returned up to the ejection pipe 31' in the reactor tank 1' via the liquid circulating lines GL3 and GL2 in the same manner as in FIGS. 14 and 16. A switching valve can be arranged appropriately in the middle of a line through which the circulated liquid moves, or at a point of merger of the flows or at a branch point so that it becomes possible to control the flow of the circulated liquid. As for the amount of the circulated liquid that moves from the reactor tank 1 to the reactor tank 1', this amount to be moved can be controlled under the assumption that the liquid 30 is sufficiently secured within the reactor tank 1.

As for the circulated gas in the reactor tank 1', FIG. 18 shows that the gas circulating line D2 is introduced to the liquid 30' in the liquid storage unit 3'. This is because almost no carbon dioxide gas remains in the treated gas after treatment in the later stage, and thus, the dissolving of carbon dioxide in the circulated liquid can be ignored even when the circulated gas is introduced into the liquid 30', and in addition, the function of stirring the liquid 30' can be added.

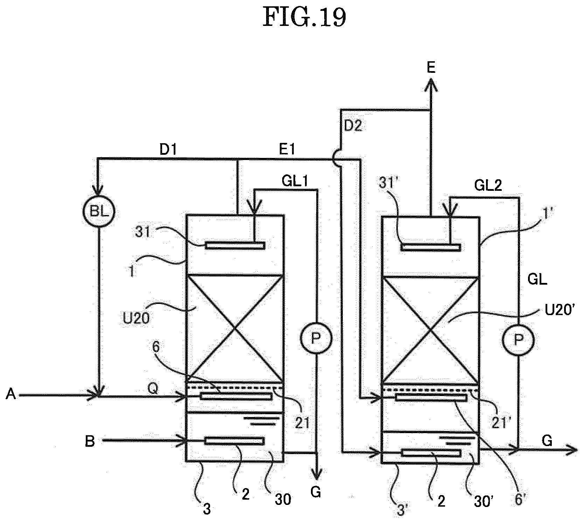

FIG. 19 shows an example of application of the methane gas generating device shown in FIG. 18 where the configuration allows the treated gas that has been treated in the reactor tank 1' to be circulated only through the reactor tank 1' by means of the gas circulating line D2. Though the route of the circulated gas is the same as that in FIG. 17, the route of the circulated liquid is different in the point where the configuration allows circulation for each reactor tank without a route that moves the circulated liquid from the reactor tank 1 to the reactor tank 1', or a route that moves the circulated liquid from the reactor tank 1' to the reactor tank 1 as in FIG.17. The configuration in FIG. 19 makes it easier to optimally adjust the liquid state within each reactor tank. Of course, it is possible to set the routes for the circulated liquid as shown in FIG. 18 in advance, where a control valve is provided for each route instead of providing the routes for the circulated liquid as shown in FIG. 19 so that the routes can be switched to be the same as in FIG. 19 at the time of use. It is possible to apply this configuration in the same manner for the circulated gas.

Next, the experiment results are shown on the basis of an embodiment where the device for generating methane gas shown in FIG. 4 was used and a comparative example shown in FIG. 2. The embodiment and the comparative example are provided in order to understand the present invention and the advantages thereof better, and thus, the present invention is not limited to the contents of the embodiment.

### (Conditions for Embodiment)

The carrier filled layer (fixed bed) was started up with digested sludge being used as seed sludge to be attached to the carrier. The treatment temperature was set at 55 °C.

The height of the carrier filled layer was 1 m and the filled volume was 10 L.

The used carrier was in a columnar shape (hollow) with the outer diameter being 10 mm, the height being 10 mm, and the specific surface area being 1000 m²/m³.

The flow amount ratio of hydrogen gas to carbon dioxide gas was set at approximately 4: 1.

The circulated liquid flow amount was 4 L/h.

The circulated gas flow amount was 3 L/min, and as for the circulated gas amount relative to the supply amount of the injected gas (carbon dioxide gas and hydrogen gas), the circulated gas was approximately 7 times greater than the injected gas (circulated gas magnification).

The injected hydrogen gas load HLR was 50 L_{H2}/L-Reactor/d, and the injected mixed gas amount was 62.5 L/L-Reactor/d.

Continuous treatment was carried out over approximately six months under the above-described conditions.

In this experiment, the magnification of the circulated gas relative to the injected gas (carbon dioxide gas and hydrogen gas) was varied from 0.2 to 50 when the change in the methane conversion ratio was also examined. In addition, the methane conversion ratio at the time when the magnification was 50 or greater was examined.

### (Results of Embodiment)

As a result of the above, the methane conversion ratio gained in the anaerobic bioreactor tank was approximately 90 % or higher and the CH₄ concentration in the generated gas gained in the reactor tank was approximately 70 % or greater.

When the circulated gas magnification was 0.2, which is lower than 0.5, the methane conversion ratio had a value that was as low as 50 % or less. Meanwhile, when the circulated gas magnification was 0.5 to 2, the gained methane conversion ratio was 70 % to 80 %. In the case where the circulated gas magnification was 2 to 20, the gained methane conversion ratio was 80 % to 95 %. Furthermore, in the case where the circulated gas magnification was 20 or greater, a stable treatment performance where the methane conversion ratio was always 95% or higher was achieved. When the circulated gas magnification was 50 or greater, however, the methane conversion ratio was lowered to less than 70 %.

In contrast, the bioreactor tank shown in FIG. 2 was used in the comparative example, and treatment was carried out under the following conditions. No circulated gas was provided in the comparative example, where H₂ and CO₂ were injected directly from beneath the carrier filled layer (fixed bed). In addition, the liquid was circulated from the liquid storage unit 3 and was supplied from the upper portion of the carrier filled layer (fixed bed), where the supply amount was 0.5 L/h, which was as small as 1/8 of that in Embodiment 1. The conditions were the same as in Embodiment 1 in terms of the used carrier and the filled carrier volume.

### (Conditions for Comparative Example)

The carrier filled layer (fixed bed) was started up with digested sludge being used as seed sludge to be attached to the carrier. The treatment temperature was set at 55 °C.

The height of the carrier filled layer was 1 m and the filled volume was 10 L.

The used carrier was in a columnar shape (hollow) with the outer diameter being 10 mm, the height being 10 mm, and the specific surface area being 1000 m²/m³.

The flow amount ratio of hydrogen gas to carbon dioxide gas was set at approximately 4: 1.

The circulated liquid flow amount was 0.5 L/h.

The injected hydrogen gas load HLR was 50 L_{H2}/L-Reactor/d, and the injected mixed gas amount was 62.5 L/L-Reactor/d.

Continuous treatment was carried out over approximately six months under the above-described conditions.

### (Results of Comparative Example)

As a result of the above, the methane conversion ratio was as low as approximately 65 %, and the CH₄ concentration was as low as approximately 30%, and thus, the exhibited values were significantly lower than those in the embodiment.

### Industrial Applicability

As described above, the present invention can provide a device and a method for generating methane gas that involves the removal of carbon dioxide where generation of high concentration methane gas is possible with high efficiency even in the case where a microorganism is used.

### Reference Signs List

- 1: Anaerobic bioreactor tank
- 2, 6: Air diffusing means
- 3: Liquid storage unit
- 4: NH₃ removing means (Removal device)
- 5: Gas separating means (Separation device)
- 20: Fixed bed
- A: Carbon dioxide containing gas
- B: Hydrogen containing gas
- D: Circulated gas
- E: Treated gas

## Claims

1. A device for generating methane gas that involves the removal of carbon dioxide where a hydrogen containing gas and a CO₂ containing gas are supplied to an anaerobic bioreactor tank so that microbial treatment is carried out to discharge a treated gas that contains methane gas, **characterized in that**
a fixed bed that is filled in with a carrier to which microorganisms are attached is provided within the reactor tank, and
a gas circulating line is provided to circulate at least a portion of the treated gas to the reactor tank.

2. The device for generating methane gas that involves the removal of carbon dioxide according to claim 1, **characterized by** comprising:
a liquid supplying means for supplying a liquid that contains a nutrition source for the microorganisms from an upper portion of the fixed bed;
a liquid storage unit for storing the liquid that is arranged in a lower portion of the fixed bed; and
a liquid circulating line for circulating a portion of the liquid that has been stored in the liquid storage unit to the liquid supplying unit.

3. The device for generating methane gas that involves the removal of carbon dioxide according to claim 2, **characterized in that**
hydrogen containing gas is supplied to the liquid storage unit, and
CO₂ containing gas is supplied to a space between the fixed bed and the liquid storage unit.

4. The device for generating methane gas that involves the removal of carbon dioxide according to claim 3, **characterized in that** the gas circulating line is connected to the space between the fixed bed and the liquid storage unit.

5. The device for generating methane gas that involves the removal of carbon dioxide according to claim 4, **characterized in that**
the fixed bed is formed of a plurality of fixed bed units, and
the gas circulating line allows at least a portion of the treated gas to be led out from a portion that connects a certain fixed bed unit to the next fixed bed unit.

6. The device for generating methane gas that involves the removal of carbon dioxide according to any of claims 1 through 5, **characterized by** comprising an NH₃ removing means for removing NH₃ that is contained in the treated gas at a later stage of the reactor tank.

7. The device for generating methane gas that involves the removal of carbon dioxide according to any of claims 1 through 5, **characterized in that**
a gas separating means for separating methane gas that is contained in the treated gas is provided at a later stage of the reactor tank, and
the remaining gas from which methane gas has been removed by means of the gas separating means is supplied to the gas circulating line.

8. The device for generating methane gas that involves the removal of carbon dioxide according to claim 6, **characterized in that**
a gas separating means for separating methane gas that is contained in the gas that has passed through the NH₃ removing means is provided at a later stage of the NH₃ removing means, and
the remaining gas from which methane gas has been removed by the gas separating means is supplied to the gas circulating line.

9. The device for generating methane gas that involves the removal of carbon dioxide according to any of claims 2 through 5, **characterized by** comprising a means for supplying an alkali chemical in the liquid storage unit or in the middle of the liquid circulating line.

10. The device for generating methane gas that involves the removal of carbon dioxide according to any of claims 1 through 4, **characterized in that** the height of the fixed bed is set in a range from 1 m to 10 m.

11. The device for generating methane gas that involves the removal of carbon dioxide according to any of claims 1 through 5, **characterized by** comprising a circulation amount adjustment means for setting the amount of circulation of the treated gas that is to be circulated through the gas circulating line is set to 0.5 to 50 times the total amount of the hydrogen containing gas and the CO₂ containing gas that are supplied to the reactor tank.

12. A method for generating methane gas that involves the removal of carbon dioxide, where a hydrogen containing gas and a CO₂ containing gas are supplied to an anaerobic bioreactor tank so that microbial treatment can be carried out within the reactor tank so as to discharge a treated gas that contains methane gas, **characterized in that**
a fixed bed that is filled in with a carrier to which microorganisms are attached is provided within the reactor tank, and
at least a portion of the treated gas is circulated to the reactor tank.

13. The method for generating methane gas that involves the removal of carbon dioxide according to claim 12, **characterized in that** a liquid that contains a nutrition source for the microorganisms is supplied from an upper portion of the fixed bed, the liquid is stored in a lower portion of the fixed bed, and a portion of the stored liquid is circulated so as to be supplied to an upper portion of the fixed bed.

14. The method for generating methane gas that involves the removal of carbon dioxide according to claim 13, **characterized in that**
hydrogen containing gas is supplied into the stored liquid, and
CO₂ containing gas is supplied to a space between the fixed bed and the stored liquid.

15. The method for generating methane gas that involves the removal of carbon dioxide according to claim 13, **characterized in that** the treated gas that is to be circulated is supplied to a space between the fixed bed and the stored liquid.

16. The method for generating methane gas that involves the removal of carbon dioxide according to any of claims 12 through 15, **characterized in that** NH₃ that is contained in the treated gas is removed.

17. The method for generating methane gas that involves the removal of carbon dioxide according to any of claims 12 through 15, **characterized in that**
methane gas is separated from the treated gas, and
the remaining gas from which the methane gas has been removed is circulated to the reactor tank.

18. The method for generating methane gas that involves the removal of carbon dioxide according to claim 16, **characterized in that**
methane gas is separated from the treated gas from which NH₃ has been removed, and
the remaining gas from which the methane gas has been removed is circulated to the reactor tank.

19. The method for generating methane gas that involves the removal of carbon dioxide according to any of claims 12 through 15, **characterized in that** an alkali chemical is supplied to either the stored liquid or the circulated liquid.

20. The method for generating methane gas that involves the removal of carbon dioxide according to any of claims 12 through 15, **characterized in that** the ratio of the mass of H₂ in the hydrogen containing gas to be supplied to the anaerobic bioreactor tank to the mass of CO₂ in the CO₂ containing gas is set to be in a range from 1:5 to 1:11.
